# EUROPEAN PATENT SPECIFICATION

(11) **EP 2 401 684 B1**
(45) Date of publication and mention of the grant of the patent: **30.10.2019**
(21) Application number: 10746836.5
(22) Date of filing: 25.02.2010
(51) Int. Cl.: G16H 40/67, G16H 20/13

(54) **Method and system for remote monitoring and interacting with a remote device**
Verfahren und System zur überwachung eines entfernten gerätes und zur interaction mit diesem
Procédé et système pour surveiller et interagir avec un dispositif à distance

(30) Priority: 25.02.2009 US 208628 P
(43) Date of publication of application: 04.01.2012
(73) Proprietor: Medicasafe, Inc., New York, NY 10013 (US)
(72) Inventor: ERVIN, Matthew, J., New York, NY 10013 (US)
(74) Representative: 2s-ip Schramm Schneider Bertagnoll Patent- und Rechtsanwälte Part mbB
(86) International application number: PCT/US2010/025422
(87) International publication number: WO 2010/099324

(56) References cited:
- US-A1- 2006 149 323
- US-A1- 2006 218 011
- US-A1- 2006 259 587
- US-A1- 2008 228 317
- US-B1- 6 751 730

## Description

### BACKGROUND OF INVENTION

Many devices and machines are in need of monitoring and management via ongoing interaction. In the healthcare domain, many medical devices, such as pill containers, medication dispensers, or glucose monitors, are provided to patients for use in an outpatient setting, yet healthcare providers desire some ability to monitor and interact with these devices. Information about when medication is taken, or when glucose levels are monitored, or measurements of glucose levels, may be known to patients but in practice this information is rarely communicated back in a timely or accurate manner to physicians or other healthcare workers. Instead, healthcare providers typically gather information when the patient returns to a medical setting. It would be medically valuable to gain insight into patient symptoms, medication usage, and other medically relevant metrics between doctor visits. There currently exists no practical technique for gathering this information. Similarly, for devices used in other domains, there are challenges to implementing practical means of monitoring and remotely interacting with the devices.

One potential option is to develop devices that can gather and communicate relevant data electronically, perhaps via a wireless modem. Unfortunately the cost of wireless modem technologies typically makes this technique prohibitively expensive, and these technologies are also subject to error. Another technique is to provide the users with devices that store information and can be interrogated in some manner when the device is returned, for example interrogated by a healthcare worker when the device is returned to a medical setting. This approach does not allow for the gathering of actionable data between patient visits. Another technique is to rely on the user to voluntary communicate information. Unfortunately, people are prone to error and may report information incorrectly, or selectively. In the case of medical information, healthcare professionals may have a difficult time making critical medical decisions based purely on patient representations of the facts. Studies suggest a high level of patient fallibility in reporting information.

The invention described herein provides a solution to the dilemma described above. This invention enables the gathering of useful information from a remote party, for example a patient, at an affordable cost. Moreover, this invention enables that information to be gathered from a device in a manner that virtually assures its accuracy. This invention also enables the verifiable dissemination of information to the device even though the device user may be unreliable.

The invention described herein was conceived for use in a healthcare setting. But it has applicability in other settings, such as the monitoring of weapons, devices with dangerous qualities, or the monitoring of devices controlled by users prone to undesirable behavior or error.

US 2008/228317 A1 relates to a method, system, and apparatus for controlling patient access to medicaments. The method, system and apparatus can include an interfacing system that can receive identifying data from a user of a medicament dispensing device. The interfacing system can also communicate limited-use access authorization data to the user via a communicative intermediary. The limited-use access authorization data enables the dispensing device to dispense a medicament up to a certain limit in response to entering the access authorization data into the dispensing device. The dispensing device may not be connected to a network, but is capable of computing and storing a limited-use access authorization data as well as validating an inputted access authorization data. ,However, the access authorization data is not described to comprise device instructions for re-mote programming of the device.

### SUMMARY

It is an object to provide a method, and system for managing device data for controlling a medication dispenser device which allows changing
the treatment implementation remotely in a more sophisticated and secure way, but nevertheless easy for the user.

This object is solved by the method, and system according to the respective independent claims.

Advantageous embodiments of the invention are defined in the respective dependent claims.

This invention relates to monitoring and remotely interacting with a device. For example, the device could be a pill container tracking pills dispensed by the user.

The device may be implemented with a processor and a display, to communicate a dynamic status code to the device user. This status code is typically encoded in a format that appears random to the user and is hard to guess or falsify. This status code could be, for example, a long number shown on the device's display that changes as device status changes. Typically only a small subset of the possible numbers may be valid status codes at any one time.

Device data can be encoded by the device processor into a dynamic status code via a variety of algorithms. For example, a device that dispenses pills may log when pills are dispensed, and an algorithm will be used to encode a summary of this dispensing history into a single 9-digit status code.

This invention enables useful device information to be relayed to a master system, usually a computer, without a data connection between the device and the master system. Instead, the user is instructed to 'check-in' and relay the device status data to a master system, typically a remote computer system, using whatever communications channel is available and most practical. For example, the user can use a telephone or an internet-connected computer to relay the status code. The user may be instructed to check in periodically, perhaps weekly, or may be prompted to check in when alerted. The alert could be a beeping sound on the device, or a variety of externally generated alerts such as calling the patient or sending a reminder email.

According to an embodiment of the invention the device status code can be encoded or encrypted in such a manner that it is difficult for the user to falsify. Thus, when the remote master system receives a valid status code from the user, it can be confident of the accuracy and reliability of the device data being transmitted.

The device status code can be decoded by the master system to reveal information about the device or the device usage. For example, one status code might indicate "device readings are normal," while a different status code might indicate "there is a problem," and the master system can derive these meanings from the status data because both the device and the master system are programmed with a parallel algorithm to allow the understanding and interpretation of the various codes. For example, if a user is prescribed to take medication twice per day from a handheld medication dispensing device, yet the user fails to take any medication, the dispenser could display a number as the status code that, when relayed and decoded during check-in, indicates to the master system that no pills have been taken and the master system can therefore initiate a medical response. If, instead, the user takes medication twice per day, a different number should be displayed by the device as the status code and no medical response will be necessary.

During the check-in process, the master system can also respond with information for the user. This may be instructions, advice, or data to input into the device. Data to input can be an access code that modifies or regulates the functioning of the device. For example, the user might be told an 'access code' during a telephone call, a number that the user is instructed to enter into the device via the device keypad. The access code can be a number that is encoded and encrypted in a manner that makes it hard to falsify. Hence, the user can only input valid data into the device if it is received from the master system and is properly encoded and valid.

Various algorithms can be used to encode and decode status codes or access codes for a particular device. Codes can be pseudo-randomized to make the codes opaque to the user and unique to the device or user. For example, the binary representation of the status code mentioned above could be XORed ("Exclusive Or" is a well known logical operation) against device-specific rolling codes, such that the resultant status code always appears random and unique for each device and for each status.

Valid device status data can be made hard to guess. If, for example, a device status code is displayed to a user as a 9 digit number, and only 10,000 such numbers are valid at any one time, and the user has no knowledge of which 10,000 numbers are valid, then a user would only have a 1 in a 10,000 chance of guessing a valid status code. Thus the master system can be reasonably confident that any valid status code received from a user is accurate status data for the device, and any information decoded from this status data can be considered reliable and accurate. This is desirable, as otherwise information received from device users is often inaccurate and misleading.

A master system can also encode any information to be entered by the user into the device: an access code, instructions, etc. An encoding algorithm, including encryption via a rolling codes or any other encryption technique, can create a set of valid input codes that appear to be a random subset of a larger set of possible codes. Thus, as with status codes, input codes can be made hard to guess, attempts to input data incorrectly will usually be interpreted by the device as 'invalid' data, and any valid input code entered into the device can be trusted as accurate information relayed from the master system. This is desirable, as instructing users to directly change the settings of a device can result in undesirable settings as a result of incorrect user behavior, either unintentional or intentional.

Using encoded data, the device and master system can verifiably communicate, with the user serving as a relay. If a user fails at an expected task, for example if the user fails to check-in at an expected time, then the master system can note this user failure and can take action to address the issue, alert third parties, etc.

### BRIEF DESCRIPTION OF THE DRAWING FIGURES

The present invention will be more readily understood from the detailed description of exemplary embodiments presented below considered in conjunction with the accompanying drawings, in which:
Figure 1 is an exemplary diagram of the relationship between major components of the invention such as a device and a master system;
Figure 2 is an exemplary diagram illustrating the fundamental components of a device;
Figure 3 is an illustration of an exemplary embodiment of a device with the components necessary for proper functioning of the system;
Figure 4 is an illustration with exploded detail of the exemplary device;
Figure 5 is an illustration providing an alternative perspective of the controlling unit of the exemplary device;
Figure 6 is an illustration of a component of the exemplary device;
Figure 7 is an exemplary flow diagram illustrating the check-in process between a user and a master system;
Figure 8 is an exemplary flow diagram illustrating the input of an access code into the exemplary device;
Figure 9 is an exemplary report resulting from the check-in process.

### DETAILED DESCRIPTION

Aspects of the invention are disclosed in the following description and related drawings directed to specific embodiments of the invention. Additionally, well-known elements of exemplary embodiments of the invention will not be described in detail or will be omitted so as not to obscure the relevant details of the invention. Further, to facilitate an understanding of the description, discussion of several terms used herein follows.

The word "exemplary" is used herein to mean "serving as an example, instance, or illustration." Any embodiment described herein as "exemplary" is not necessarily to be construed as preferred or advantageous over other embodiments. Likewise, the term "embodiments of the invention" does not require that all embodiments of the invention include the discussed feature, advantage or mode of operation.

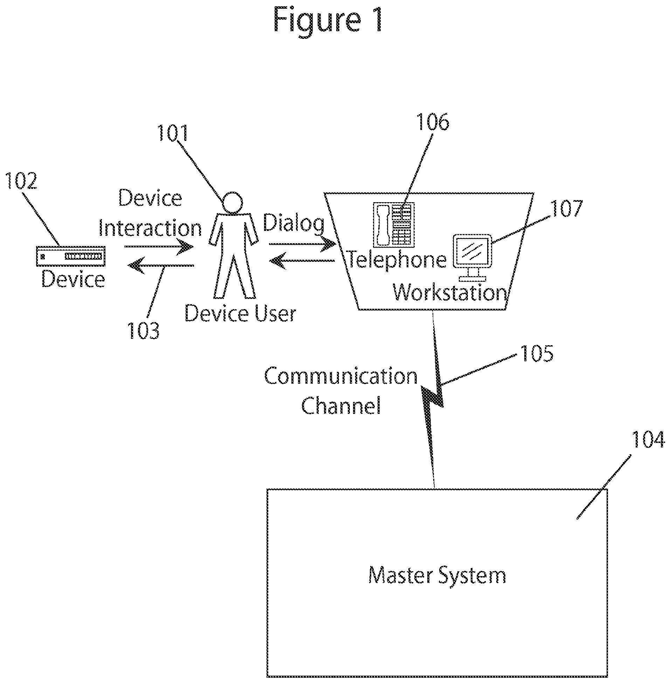

Figure 1 illustrates an embodiment of the present invention. A device user 101 has control of device 102, a device that can collect useful information. The invention relates to the interaction between this device 102 and the master system 104, an interaction which is mediated by the user 101 using a communication channel 105.

The device 102 should include a means to communicate with a device user 101, typically a display or a speaker. The device 102 should be able to communicate a status code via this display or speaker. This aspect is shown in figure 1 as interaction 103 between the user 101 and the device 102. The status code could be, as a non-limiting example, a dynamically generated number, like 209302939, which from the point of view of the device user 101 would appear to be a seemingly random number. The status code could also take a variety of other formats, such as an alphanumeric format, a visual symbol, a sound, or any other format used to communicate information from the device 102 to a user 101.

The device user 101 is expected to 'check in' by phone 106 or by internet-enabled computer 107, or by whatever communications means 105 is convenient. 'Check-in' will involve communicating the current device status code displayed (or communicated by any means).

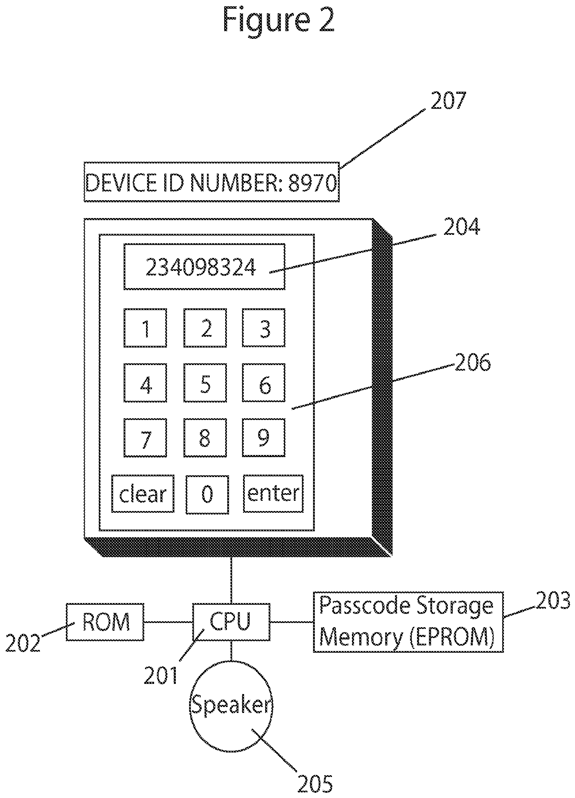

Figure 2 is a diagram of an exemplary implementation of the user device 102. A central processing unit 201 is augmented with ancillary components for storing programmatic instructions and data, such as read-only memory 202 or read/write storage memory 203. The exemplary device 102 also includes some degree of output capability, typically a display 204 or a speaker 205, and may enable user input, typically via a keypad 206. The device 102 may have information printed upon it, such as a device identifying number 207.

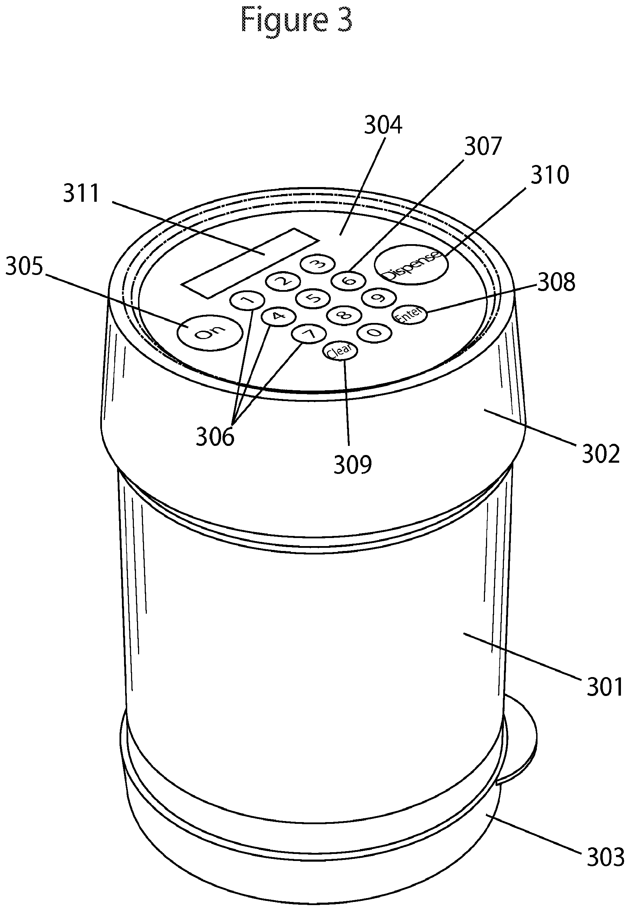

A non-limiting exemplary embodiment of a device 102 is shown in Figures 3 through 6. This exemplary device embodiment is a medication dispenser. Figure 3 shows that the medication dispenser contains a pill tray stack 301 which contains pills. The pill tray stack 301 is moved by a controller unit 302 which contains microcontroller electronics, schematically similar to Figure 2. The controller unit 302 also contains a mechanical motor which can be programmatically activated by the central processing unit to rotate the top tray on the pill tray stack 301. As the controller unit 302 rotates the top pill tray in the stack, pills from that tray can fall to the bottom of the dispenser, and removing the bottom cap 303 enables a user 101 to retrieve the pills. The user 101 interacts with the controller unit 302 via a control panel and display 304 which contains an "On" button 305, a standard keypad 306 with digits 307, an "Enter" key 308, and a clear key 309. The controller panel also contains a "Dispense" button 310. Information, including the device status data, can be read by the user 101 via display 311.

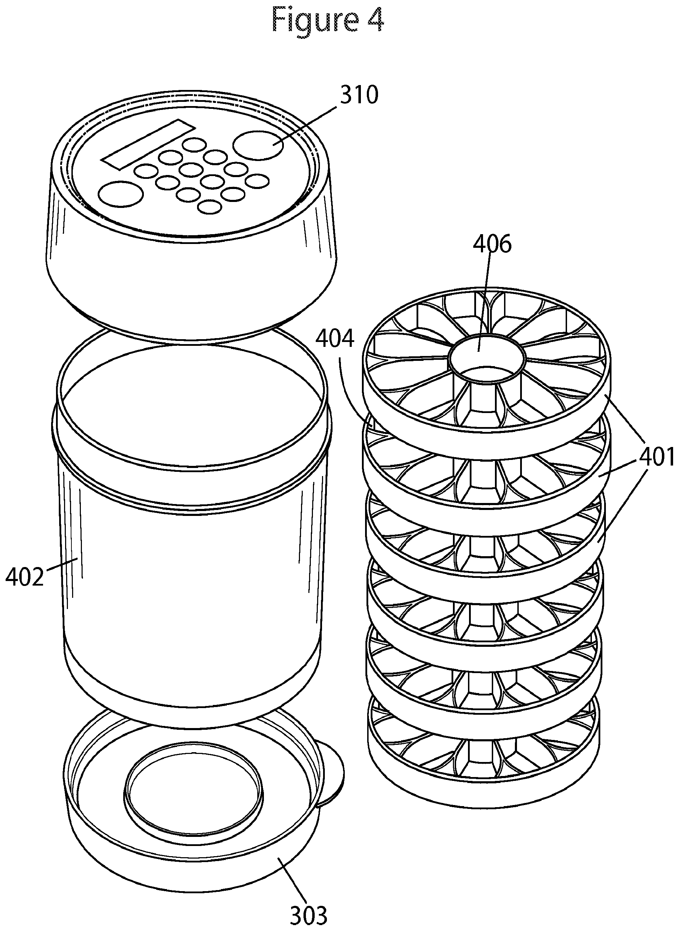

Figure 4 shows exploded details of the dispenser, including more details of the trays 401, which are within ajar 402. If the user 101 presses the dispense button 310, the controller unit logic may deem it appropriate to dispense a pill, and if so the controller unit 302 will move the first tray such that a pill can drop down pill slot 404. Each tray 401 has a slot, and at initiation all slots are aligned, such that a pill from the top tray will drop down this slot when moved into position. The user 101 can remove cap 303 to obtain the pill.

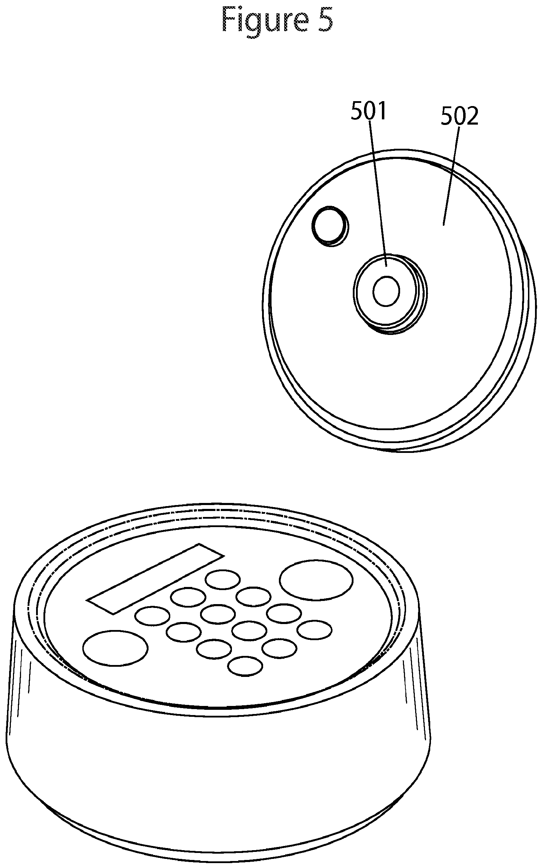

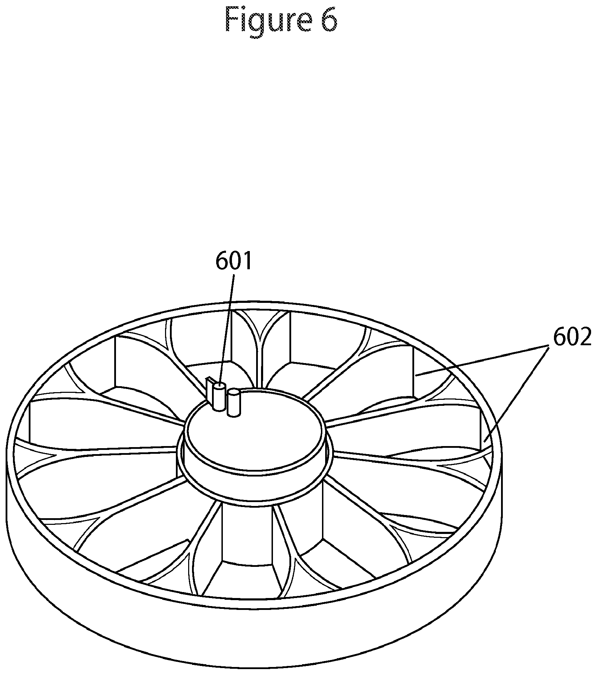

Figure 5 shows the underside of the controller unit 302. The underside is rotated by a motor within the control unit 302, and thus rotates a drive post 501 protruding from a drive plate 502. This drive post 501 mates into the drive lug 406 protruding from the inside ring of the top tray. Thus the top tray is rotated by this drive post 501. Moreover, the underside of each tray 401, as shown in figure 6, also contains a drive post 601. Once a tray is rotated completely, its drive post 601 collides with the drive lug 406 from the tray beneath it. In this manner each tray 401 can be sequentially rotated, and each tray 401 rotates so that the pills contained in pill drawers 602 can fall down the pill slot 404.

To accomplish the invention described herein, specific techniques and details for the implementation of controller unit 302 are readily known to or can be developed by a person of ordinary skill in the art. Within this controller unit 302 is an electro-optical eye watching markings attached to the drive plate 502, and thus the microcontroller processor can note input from this sensor and track rotation of the drive plate 502. The microcontroller is programmed to stop the drive plate rotation based on signals from this electro-optical eye, and the microcontroller also keeps track of the total units of rotation, which translates to the number of pills dispensed.

In this exemplary medication dispenser, the processor 201 in the controller unit 302 also tracks time, logs the time when pills are dispensed, and effectively "locks", meaning it fails to dispense, under certain logical conditions, such as when the user 101 has already dispensed enough pills to meet the prescription.

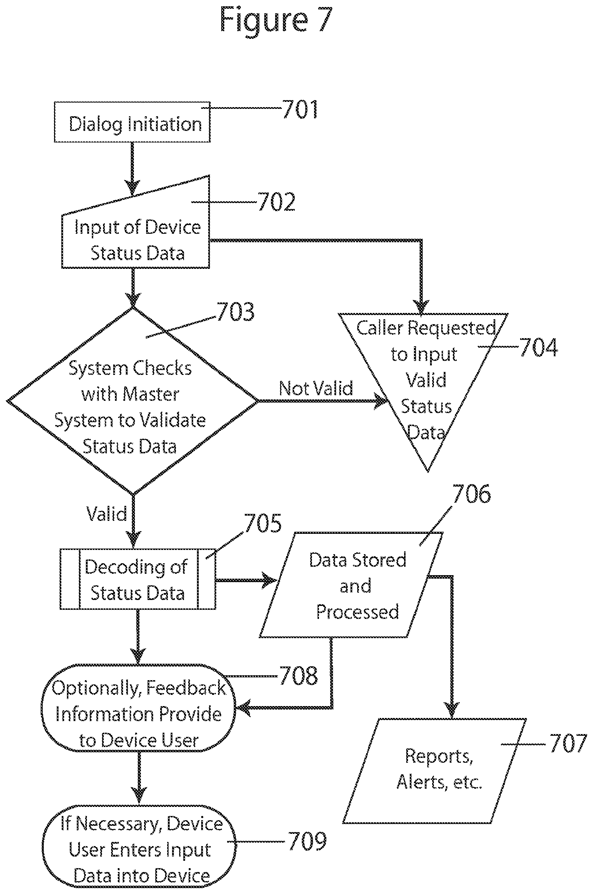

Figure 7 reflects the workflow of a 'check-in process' between a user 101 and the master system 104. During this check-in, the user 101 will be instructed to relay the device status code, and other data. In the case of the exemplary embodiment described herein, the medication dispenser, the device status code can be dynamically displayed by the device 102. The 'status' of this exemplary device, as reflected in this dynamic status code, is primarily a reflection of the recent dispensing history of the device. If many pills have been dispensed recently, the status code will be different than had the device 102 dispensed no pills recently.

The check-in process described in figure 7 can occur periodically, perhaps weekly, or may occur when the user 101 is alerted to check-in. An alert could be a beeping sound on the device 102, or a variety of externally generated alerts such as calling the patient or sending a reminder email.

During check-in, the user 101 is expected to communicate with a master system 104, typically a computer system, using whatever communications channel 105 is available and most practical. For example, the user 101 can use a telephone 106 to communicate via an Interactive Voice Response (IVR) system to the master system 104, or the user 101 can use an internet-connected computer 107 to check-in via a website.

In step 701, the user 101 initiates the check-in process by communicating with the master system 104 via communications channel 105. The check-in process will involve a dialog, and at the initial step 701 the user 101 may be asked for identifying information, such as the user's name, a user ID number, identifying information for the device 102 such as a device ID, etc. Collecting identifying information in step 701 is customary but not absolutely necessary, as the device status code, discussed below, could incorporate identifying data in an encoded format. For the medication dispenser described herein as an exemplary embodiment, step 701 entails gathering the device ID number from the user 101. This device ID number is printed on a sticker 207 that is attached to the dispensing device 102.

In step 702, the user 101 will communicate the current device status code. For the medication dispenser described herein as an exemplary embodiment, the status code can be seen by the user 101 on display 311 in response to pressing the 'On' button 305 then the 'Enter' key 308.

A dynamic device status code can be created by an algorithm which is used to encode a variety of information. For example, the status data could contain information about the times at which the device 102 was operated by the user 101, measurements collected by the device 102, etc. This information will typically be condensed by the microprocessor 201 on the device 102, using an appropriate algorithm. For example, a variety of device states can be summarized by one status code that indicates "all is well," while many different states can be summarized by a different status code that indicates "there is a problem."

In the exemplary embodiment of the medication dispenser, the status code is a number which may be 8 or 9 digits, short enough for a human to copy and relay to the master system 104 with ease. In binary representation, and in accordance with an embodiment of the present invention, the exemplary status code contains 29 bits, and information is encoded in various bits of this code. Of the 29 bits, certain groups of bits represent dispensing history data for a period of time, such as a day. For example, 14 of the 29 bits can be subdivided into 7 groups of 2 bits, each 2 bit group representing pills dispensed in a day, where there are 4 possible states: "00" represent no pills taken, "01" for 1 pill taken, "10" for 2 pills taken, and "11" for 3 or more pills taken. The time interval and pill count intervals can be reassigned at treatment initialization to be appropriate to the prescription. The remaining bits can be allocated toward 1) a pill count, which would hold an integer representation of the total number of dispenses during the check-in time period and 2) a checksum or other required data that limits the number of, or maintains the integrity of, the possible codes. For instance, 5 of the remaining bits can be used to hold an integer count of up to 32 dispenses. Some of the remaining 10 bits can contain a checksum or can contain arbitrary bits that, if not communicated properly by the user 101, will indicate that the user 101 has communicated a false or incorrect status code. For example, 4 bits can hold a checksum derived from the two's complement of the rest of the bits summed in groups of 4 bits. A checksum of 4 bits constrains the status code to 1/16^{th} of the possible array of status codes. Additional checksums or arbitrary data can be encoded into the remaining 6 bits, such that the odds of a user 101 falsifying an acceptable status code would be relatively low.

A select few such disallowed codes can be used to communicate special device states; for instance 7 days of "00" pills per day, in combination with a pill count of 32-which could not represent a true situation if "00" represents 0 pills dispensed within a window-can indicate that the device's power was reset and initialization or reinitialization of the treatment is required.

In an embodiment of the invention, to disguise the status code encoding process from the user 101, and to create seemingly random status codes for each check-in, the 29 bits can be obfuscated by performing an XOR (the logical exclusive OR operator) operation on them against a rolling set of codes that are unique to each device 102. These codes are stored both in the device's memory and the master system's memory, but are not known to the user 101. For each check-in, a different rolling code is used by both the device 102 and master system 104. With the successful completion of a check-in, the master system 104 and device 102 advance to the next rolling code. Knowing the rolling code, and the status code algorithm, the master system 104 can 'decode' the status code relayed by the user 101 in step 702, deciphering the information about the total number of pills dispensed, and the number of pills the user 101 has dispensed each day, and can create reports with this data.

The master system 104 must be tolerant of a user 101 entering in the same code twice, realizing the user's error and not advancing the rolling code. This applies equally to incomplete check-ins.

Note that since the device 102 appears to generate random eight or nine digit status codes, and since only a relatively small subset of possible status codes are valid at any one time, the user 101 will have a hard time forging misleading or incorrect data.

A user 101 could attempt to find a valid status code by relaying many codes successively to the master system 104 until one is accepted. The exemplary master system 104 is programmed to allow a limited number, for example, four successive attempts, to input invalid status codes, which suggests a brute force attack, and thereafter directs the user 101 to contact a separate authority to obtain authorization to continue with the check-in. Success attempts to check in with invalid codes can also be noted on the user's report so that the user's credibility and compliance with inputting data can be assessed.

To generalize, the master system 104 will decode valid status codes to extract data and create reports. The master system 104 is programmed with knowledge about the device's algorithm for generating status codes, and thus the master system 104 can determine if a status code is valid in step 703, and, if not, request in step 704 an accurate or valid status code. If the status data is valid, the master system 104 can be programmed to decode in step 705 some meaning from the particular status code relayed by the user 101. The master system 104 may store and process this information in step 706 further to derive additional information, and may optionally generate reports or alerts in step 707 for the user 101 or for other parties.

In the case of the exemplary medication dispenser, if the status code indicates that the appropriate number of pills have be taken at appropriate times, then the master system 104 might generate a report indicating that "all is well." If the status indicates that no pills were taken, or too many pills were taken, then a report can be generated that indicates this bad pattern and alerts healthcare workers that "there is a problem."

Depending on the nature of the specific device 102 used in conjunction with this invention, the status codes might relay an approximation of use, assessment scores, user behavior, etc. Many devices that collect information exist, and one skilled in the art can modify and program such devices to track a variety of information, encode a summary of this information into a status code via an algorithm, and program a master system 104 to decode this status code when it is relayed by the device user 101.

Common algorithmic techniques can be employed to generate device status codes such that a user 101 will have some difficulty guessing a valid status code. The status code generating algorithm could take advantage of time stamps, randomized key codes, and other techniques known to those skilled at the art. The algorithm used to generate status data can generate codes that are seemingly random to the device user 101. Moreover, the codes can be made long enough and random enough that the device user 101 will have difficulty relaying acceptable status data unless the user 101 relays the exact status data communicated to the device user 101 by the device 102. Therefore the user 101 can find it difficult to falsify status code information, or to relay false information accidentally. Thus the master system 104 can be reasonably confident that any valid status code received is the accurate status code communicated by the device to the device user 101.

In step 702 the master system 104 can also collect other information from the user 101. In the case of the medication dispenser, in step 702 the dialog asks for additional information about patient symptoms, activities, general health, and medication side effects.

The master system 104 may also respond in step 708 with information for the user 101. This information may be instructions, advice, or data to input into the device 102. In the exemplary medication dispenser device, the master system 104 responds in step 708 with an access code number and instructions for the user 101 to enter this number into the device 102 via keypad 306. The user 101 is expected to enter that access code into the device 102 in step 709.

The controller unit 302 derives programmatic instructions when a valid access code is entered into the exemplary device 102 via the keypad 306. Via the access code the controller unit 302 can be instructed to limit the total number of pills dispensable within a certain time (e.g., no more than 2 pills in 6 hours), remind the user 101 to take pills after a certain time (e.g. if the user 101 has not dispensed a pill within the prior 24 hours, remind the user 101 via beeps and messages on the display 311), remind the user 101 to check-in (e.g. if 5 days have passed, the user 101 is reminded to check-in via beeps and a message on the device display 311), and can instruct the controller unit 302 to inactivate the dispense action if the user 101 has failed to input a new access code (i.e. failed to check-in) within a certain period of time. The access code input can also inform the controller unit 302 of the total number of pills in the dispenser and can reprogram the controller unit's clock with a new time.

The algorithm for encoding and decoding the access code is known to both the master system 104 and the device 102, such that the device 102 can decode the information contained in the access code relayed by the user 101 from the master system 104.

In this exemplary embodiment, the access code is a number that is short enough for a human to easily hear over the telephone or read on a website and input into their device 102 via keypad 306 without trouble.

To maximize the utility of each access code, the exemplary algorithm encodes a variable amount of information. Information is added to the access code incrementally from lowest to highest order bit. The first six or seven bits represent the total pill count as derived by the master server in step 702 from the status code. Echoing back this pill count limits the set of valid access codes, and is information that can be used to deduce that the user 101 properly communicated the most recent status code to the master system 104.

Additional bits may be appended to the high order end of the access code number to relay instructions to the device 102 in coded format; these codes are supplied in a format of a two or three bit flag to determine the instruction type, followed by a preset number of bits per instruction type that contain the value of the instruction. Sample instructions can be: (a) set a lockout on the device after a specified number of pills per time period (e.g., after the user 101 take 3 pills in a 24 hour time period), (b) add audible daily reminders to the treatment plan, or (c) change the length of time before the access code expires and another check-in is mandatory.

Finally, bits are appended for a checksum, calculated by summing the entire access code number in groups of bits and taking the two's complement, which serves as one degree of limitation on the set of valid access codes for a check-in. The instruction bits are included in the check-sum procedure, and because they contain their own specific valid and invalid values, further restrict the probability of randomly finding a valid access code.

Similar to the status codes, an XOR between the access codes and a set of rolling codes is applied such that the valid access numbers appear entirely random to the user 101. The user 101 will find it difficult to falsify codes because there is high improbability of finding a valid code by guessing. Moreover, the device 102 only allows a small number of invalid entry attempts before initiating a lockout that prevents further attempts for a period of time, thus discouraging the user 101 from attempting to brute-force the access code with multiple successive entries.

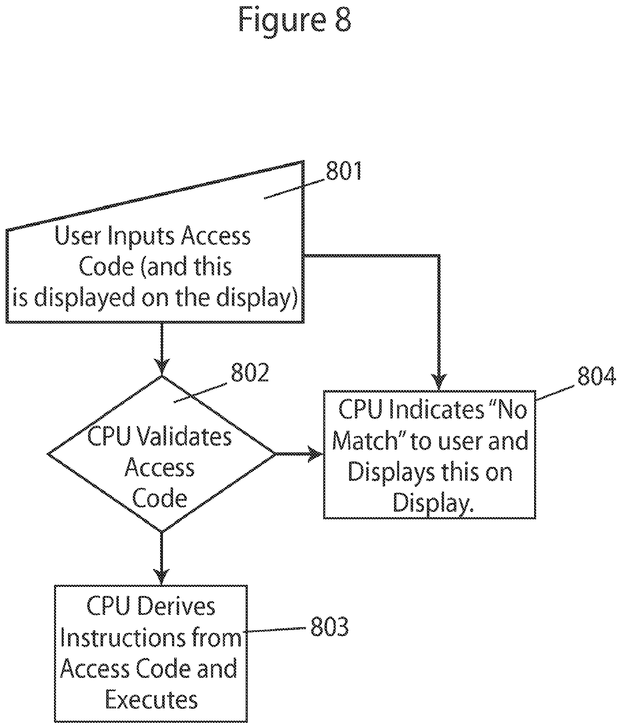

Figure 8 illustrates the entry of access codes. The user 101 inputs the access code into the device 102 in step 801. The device 102 checks the validity of the code in step 802 and, if it is valid, interprets the instructions contained within in step 803. Otherwise, the device 102 signals to the user 101 that the access code input is invalid in step 804.

The access code for initialization will usually be long, as it contains flags for instructions on all of the details about the treatment regimen. Subsequent access codes will usually be relatively short, as most details of the treatment do not change.

The ability for the master server to insert device instructions into each access code facilitates remote programming of the device 102, which potentially allows for a complex treatment plan to be executed with little intervention on the part of a primary care physician or researcher. Automated logic can evaluate the information supplied by a patient during the check-in process, then alter the treatment and reprogram the medication dispenser accordingly via relay of an access code containing the desired instructions.

To generalize, an access code can be relayed to the device 102 via the user 101, and that access code can modify the behavior of a device 102, can modify the data stored in the device 102, can modify the mode of data collection used by device 102, etc. This access code can be encrypted and sufficiently random such that the user 101 would not be able to easily guess a valid access code. The device 102 can be programmed by a person of ordinary skill in the art to decode the access code and derive the instructions. As such, the master system 104 would be able to communicate information to the device 102 with a good deal of confidence that the user 101 could not corrupt the message, whether intentionally or unintentionally, by falsifying valid input data.

At any point during the check-in workflow described in Figure 7, for example during steps 702 or 708, the dialog between the user 101 and the master system 104 may be optionally augmented to ask and ascertain additional data. For example, in the context of a medical device, the user 101 may also be asked about symptoms, activities, behaviors, attitudes, or various other issues related to health. This invention prompts a dialog between a user 101 and a master system 104, and that dialog presents an opportunity to collect valuable information and process that information in a timely manner.

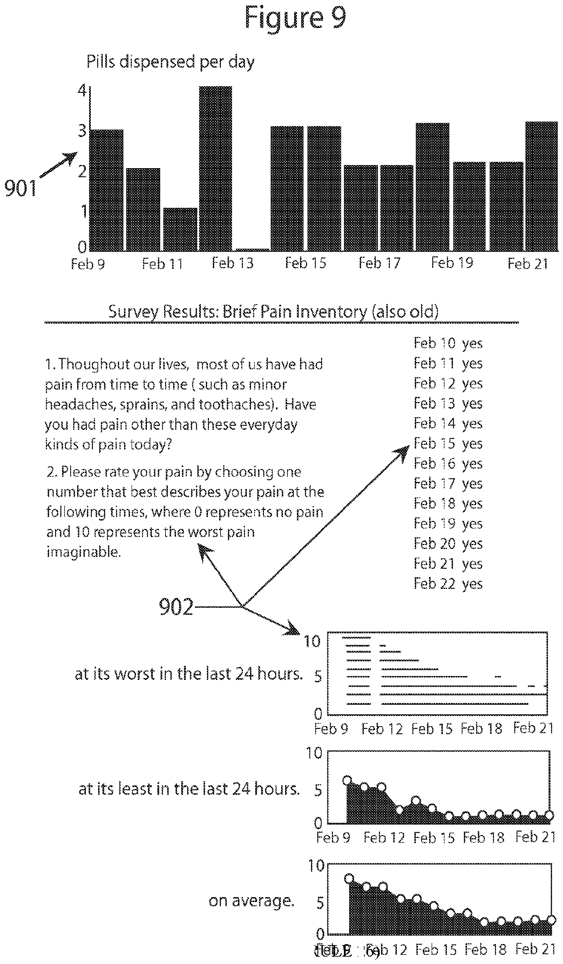

Figure 9 shows a report related to the exemplary embodiment of the medication dispenser. This report shows dispensing data in a graph 901 derived from status codes received via the check-ins. This report also graphs the user's response to other questions 902 asked during the check-in process.

If a user 101 fails to check-in at an expected time, this failure to communicate can be noted by the master system 104. The device user 101 may then be contacted proactively, or alerted to make contact with the master system 104.

If the user 101 fails to input an access code into the device 102 as instructed, the status code received from a subsequent check-in can contain a code that indicates the last access code entered, and therefore the master system 104 can be made aware of the user's failure to properly enter an access code. In the case of the exemplary medication dispenser, certain bits of the status code will not reset unless a new access code has been entered. Hence when the master system 104 receives a status code that contains a duplicate of these bits, the master system 104 can conclude that the previously communicated access code was not input into the device 102 by the user 101.

Clear benefits are derived from the exemplary medication dispenser embodiment of the invention. Healthcare professionals can be confident that a user 101 has accurately communicated data about the quantity and times at which pills were dispensed. Moreover, the master system 104 can return access codes for the device 102 which limit medication access to appropriate levels, or initiate reminders at appropriate times. Patients are known to be error prone, and the invention described herein can minimize mistaken communication.

## Claims

1. A method for managing device data, the method comprising the following steps:
alerting a user (101) of a medication dispensing device (102) to communicate with a master system (104);
generating, by the medication dispensing device (102), a status code based on device data indicating the status of the medication dispensing device (102), the status code encoding the device data;
displaying the status code to the user (101);
receiving an access code entered by the user (101) through an input pad (206, 306) of the device (102), the access code being generated by the master system (104) in response to receiving the status code via a communication channel (105), and provided to the user (101) via the communication channel (105), the access code comprising device instructions for remote programming of the device (102);
generating, by the master system (104), a report based on the status of the device encoded in the status code;
checking validity of the access code, and if the access code is valid, interpreting the instructions to reprogram the device (102), otherwise signaling to the user (101) that the access code is invalid.

2. The method for managing device data of claim 1, wherein the alerting step comprises the step of:
generating an audio alert or displaying the alert on a display of the device (102).

3. The method for managing device data of claim 1, wherein the generating step comprises:
generating a value indicative of the status of the device (102); and
performing an XOR operation on the value against a rolling set of codes.

4. The method for managing device data of one of the preceding claims, wherein the instructions comprise at least one of:
limiting the total number of pills to be dispensable within a certain time, reminding the user (101) to take pills after a certain time, reminding the user (101) to check in, and
inactivating the dispense action if the user (101) has failed to input a new access code.

5. A system for managing device data, the system including:
a medication dispensing device (102) comprising:
a device identification number;
an indicator configured to alert a user (101) to communicate with a master system (104);
a display unit (204) for displaying a status code; and
a processing unit (201) configured to interface with the indicator and the display unit (204), where the processing unit (201) is configured to generate a status code based on device data indicating a status of a device (102), the status code encoding the device data; and
the master system (104) being configured to receive via a communication channel (105) the status code from a user (101) of the device (102), where the master system (104) is adapted for generating a report based on the status of the device encoded in the status code, and for generating, in response to receiving the status code, an access code, the access code comprising device instructions for remote programming of the device (102), and for providing the access code to the user (101) via the communication channel (105);
wherein the medication dispensing device (102) further comprises:
an input pad (206, 306),
wherein the processing unit (201) is further configured to receive the access code entered by the user (101) through the input pad (206, 306), to check the validity of the access code, and if the access code is valid, to interpret the instructions to reprogram the medical dispensing device (102), otherwise to signal to the user (101) that the access code is invalid.

6. The system for managing device data of claim 5, wherein the input pad (206, 306) is further is configured to interface with the processing unit (201), and where the processing unit (201) is adapted to generate a second status code based on the access code; and
wherein the indicator is further configured to generate an audible alert or a visual alert.

7. The system for managing device data of claim 5 or 6, wherein the instructions comprise at least one of:
limiting the total number of pills to be dispensable within a certain time, reminding the user (101) to take pills after a certain time, reminding the user (101) to check in, and inactivating the dispense action if the user (101) has failed to input a new access code.

## Patentansprüche

1. Verfahren zum Verwalten von Vorrichtungsdaten, wobei das Verfahren die folgenden Schritte umfasst:
Warnen eines Benutzers (101) einer Medikamentenabgabevorrichtung (102), um mit einem Mastersystem (104) zu kommunizieren;
Erzeugen eines Zustandscodes durch die Medikamentenabgabevorrichtung (102) basierend auf Vorrichtungsdaten, die den Zustand der Medikamentenabgabevorrichtung (102) angeben, wobei der Zustandscode die Vorrichtungsdaten codiert;
Anzeigen des Zustandscodes für den Benutzer (101);
Empfangen eines von dem Benutzer (101) durch ein Eingabefeld (206, 306) der Vorrichtung (102) eingetragenen Zugriffscodes, wobei der Zugriffscode durch das Mastersystem (104) als Reaktion auf ein Empfangen des Zustandscodes über einen Kommunikationskanal (105) erzeugt und dem Benutzer (101) über einen Kommunikationskanal (105) bereitgestellt wird, wobei der Zugriffscode Vorrichtungsanweisungen zum entfernten Programmieren der Vorrichtung (102) umfasst;
Erzeugen eines Berichts basierend auf dem in dem Zustandscode codierten Zustand durch das Mastersystem (104);
Überprüfen einer Gültigkeit des Zugriffscodes und, falls der Zugriffscode gültig ist, Interpretieren der Anweisungen, um die Vorrichtung (102) umzuprogrammieren, andernfalls Signalisieren an den Benutzer (101), dass der Zugriffscode ungültig ist.

2. Verfahren zum Verwalten von Vorrichtungsdaten nach Anspruch 1, wobei der Warnungsschritt den folgenden Schritt umfasst:
Erzeugen einer Audiowarnung oder Anzeigen der Warnung auf einer Anzeige der Vorrichtung (102).

3. Verfahren zum Verwalten von Vorrichtungsdaten nach Anspruch 1, wobei der Erzeugungsschritt Folgendes umfasst:
Erzeugen eines Wertes, der den Zustand der Vorrichtung (102) angibt; und
Durchführen eines XOR-Vorgangs auf dem Wert gegen einen rollenden Codesatz.

4. Verfahren zum Verwalten von Vorrichtungsdaten nach einem der vorhergehenden Ansprüche, wobei die Anweisungen Folgendes umfassen:
Begrenzen der Gesamtzahl der Pillen, die innerhalb einer bestimmten Zeit ausgegeben werden können, Erinnern des Benutzers (101), Pillen nach einer bestimmten Zeit einzunehmen, Erinnern des Benutzers (101), einzuchecken, und/oder Deaktivieren der Abgabeaktion, falls der Benutzer (101) es versäumt hat, einen neuen Zugriffscode einzugeben.

5. System zum Verwalten von Vorrichtungsdaten, wobei das System Folgendes einschließt:
eine Medikamentenabgabevorrichtung (102), die Folgendes umfasst:
eine Vorrichtungsidentifikationsnummer;
einen Indikator, der konfiguriert ist, um einen Benutzer (101) zu warnen, um mit einem Mastersystem (104) zu kommunizieren;
eine Anzeigeeinheit (204) zum Anzeigen eines Zustandscodecodes; und
eine Verarbeitungseinheit (201), die konfiguriert ist, um sich mit dem Indikator und der Anzeigeeinheit (204) zu verbinden, wobei die Verarbeitungseinheit (201) konfiguriert ist, um einen Zustandscodecode basierend auf Vorrichtungsdaten, die einen Zustand der Vorrichtung (102) angeben, zu erzeugen, wobei der Zustandscodecode die Vorrichtungsdaten codiert; und
wobei das Mastersystem (104) konfiguriert ist, um über einen Kommunikationskanal (105) den Zustandscodecode von einem Benutzer (101) der Vorrichtung (102) zu empfangen, wobei das Mastersystem (104) zum Erzeugen eines Berichts basierend auf dem in dem Zustandscodecode codierten Zustand der Vorrichtung und zum Erzeugen, als Reaktion auf das Empfangen des Zustandscodecodes, eines Zugriffscodes konfiguriert ist, wobei der Zugriffscode Vorrichtungsanweisung für entferntes Programmieren der Vorrichtung (102) und zum Bereitstellen des Zugriffscodes an den Benutzer (101) über den Kommunikationskanal (105) umfasst;
wobei die Medikamentenabgabevorrichtung (102) ferner Folgendes umfasst:
ein Eingabefeld (206, 306), wobei die Verarbeitungseinheit (201) ferner konfiguriert ist, um den von dem Benutzer (101) durch das Eingabefeld (206, 306) eingetragenen Zugriffscode zu empfangen, um die Gültigkeit des Zugriffscodes zu überprüfen und, falls der Zugriffscode gültig ist, um die Anweisungen zu interpretieren, um die Medikamentenabgabevorrichtung (102) umzuprogrammieren, andernfalls um dem Benutzer (101) zu signalisieren, dass der Zugriffscode ungültig ist.

6. System zum Verwalten von Vorrichtungsdaten nach Anspruch 5, wobei das Eingabefeld (206, 306) ferner konfiguriert ist, um sich mit der Verarbeitungseinheit (201) zu verbinden und wobei die Verarbeitungseinheit (201) geeignet ist, um einen zweiten Zustandscodecode basierend auf dem Zugriffscode zu erzeugen; und
wobei der Indikator ferner konfiguriert ist, um eine akustische Warnung oder eine visuelle Warnung zu erzeugen.

7. System zum Verwalten von Vorrichtungsdaten nach Anspruch 5 oder 6, wobei die Anweisungen Folgendes umfassen:
Begrenzen der Gesamtzahl der Pillen, die innerhalb einer bestimmten Zeit ausgegeben werden können, Erinnern des Benutzers (101), Pillen nach einer bestimmten Zeit einzunehmen, Erinnern des Benutzers (101), einzuchecken, und/oder Deaktivieren der Abgabeaktion, falls der Benutzer (101) es versäumt hat, einen neuen Zugriffscode einzugeben.

## Revendications

1. Procédé de gestion de données de dispositif, le procédé comprenant les étapes suivantes :
alerter un utilisateur (101) d'un dispositif de distribution de médicaments (102) pour communiquer avec un système maître (104) ;
générer, par l'intermédiaire du dispositif de distribution de médicaments (102), un code d'état basé sur des données de dispositif indiquant l'état du dispositif de distribution de médicaments (102), le code d'état codant les données de dispositif ;
afficher le code d'état à l'utilisateur (101) ;
recevoir un code d'accès saisi par l'utilisateur (101) au moyen d'un clavier d'entrée (206, 306) du dispositif (102), le code d'accès étant généré par le système maître (104) en réponse à la réception du code d'état par l'intermédiaire d'un canal de communication (105), et fourni à l'utilisateur (101) par l'intermédiaire du canal de communication (105), le code d'accès comprenant des instructions de dispositif pour une programmation à distance du dispositif (102) ;
générer, par le système maître (104), un rapport basé sur l'état du dispositif codé dans le code d'état ;
vérifier la validité du code d'accès, et si le code d'accès est valide, interpréter les instructions pour reprogrammer le dispositif (102) ou, dans le cas contraire, signaler à l'utilisateur (101) que le code d'accès est invalide.

2. Procédé de gestion de données de dispositif selon la revendication 1, dans lequel l'étape d'alerte comprend l'étape suivante :
générer une alerte audio ou afficher l'alerte sur un affichage du dispositif (102).

3. Procédé de gestion de données de dispositif selon la revendication 1, dans lequel l'étape de génération consiste à :
générer une valeur indicative de l'état du dispositif (102) ; et
exécuter une opération OU exclusif sur la valeur par rapport à un ensemble de codes tournants.

4. Procédé de gestion de données de dispositif selon l'une des revendications précédentes, dans lequel les instructions comprennent au moins l'un parmi :
la limitation du nombre total de pilules à distribuer dans un certain temps, le rappel à l'utilisateur (101) de prendre des pilules après un certain temps, le rappel à l'utilisateur (101) de s'enregistrer, et la désactivation de l'action de distribution si l'utilisateur (101) n'a pas entré un nouveau code d'accès.

5. Système de gestion de données de dispositif, le système comprenant :
un dispositif de distribution de médicaments (102) comprenant :
un numéro d'identification de dispositif ;
un indicateur configuré pour alerter un utilisateur (101) afin de communiquer avec un système maître (104) ;
une unité d'affichage (204) pour afficher un code d'état ; et
une unité de traitement (201) configurée pour interfacer avec l'indicateur et l'unité d'affichage (204), l'unité de traitement (201) étant configurée pour générer un code d'état basé sur des données de dispositif indiquant un état d'un dispositif (102), le code d'état codant les données du dispositif ; et
le système maître (104) étant configuré pour recevoir, par l'intermédiaire d'un canal de communication (105), le code d'état d'un utilisateur (101) du dispositif (102), où le système maître (104) est adapté pour générer un rapport basé sur l'état du dispositif codé dans le code d'état, et pour générer, en réponse à la réception du code d'état, un code d'accès, le code d'accès comprenant des instructions de dispositif pour une programmation à distance du dispositif (102), et pour fournir le code d'accès à l'utilisateur (101) par l'intermédiaire du canal de communication (105) ;
dans lequel le dispositif de distribution de médicaments (102) comprend en outre :
un clavier d'entrée (206, 306),
dans lequel l'unité de traitement (201) est en outre configurée pour recevoir le code d'accès saisi par l'utilisateur (101) par le clavier d'entrée (206, 306) afin de vérifier la validité du code d'accès, et si le code d'accès est valide, pour interpréter les instructions de reprogrammation du dispositif distributeur de médicaments (102), dans le cas contraire, pour signaler à l'utilisateur (101) que le code d'accès est invalide.

6. Système de gestion de données de dispositif selon la revendication 5, dans lequel le clavier d'entrée (206, 306) est en outre configuré pour interfacer avec l'unité de traitement (201), et où l'unité de traitement (201) est adaptée pour générer un second code d'état basé sur le code d'accès ; et
dans lequel l'indicateur est en outre configuré pour générer une alerte audible ou visuelle.

7. Système de gestion de données de dispositif selon la revendication 5 ou 6, dans lequel les instructions comprennent au moins l'un parmi :
la limitation du nombre total de pilules à distribuer dans un certain temps, le rappel à l'utilisateur (101) de prendre des pilules après un certain temps, le rappel à l'utilisateur (101) de s'enregistrer, et la désactivation de l'action de distribution si l'utilisateur (101) n'a pas entré un nouveau code d'accès.
